(19) Europäisches Patentamt
European Patent Office
Office européen des brevets

(11) Publication number: 0 430 637 B1

(12) EUROPEAN PATENT SPECIFICATION

(45) Date of publication of patent specification: 28.09.94

(51) Int. Cl.⁵: C12N 9/50, C12N 9/54, A61K 37/54

(21) Application number: 90312855.1

(22) Date of filing: 27.11.90

(54) **Fibrinolytic protein and production method thereof.**

(30) Priority: 27.11.89 JP 308191/89

(43) Date of publication of application:
05.06.91 Bulletin 91/23

(45) Publication of the grant of the patent:
28.09.94 Bulletin 94/39

(84) Designated Contracting States:
CH DE ES FR GB IT LI

(56) References cited:
EP-A- 0 130 756

INTERNATIONAL JOURNAL OF PROTEIN RE-
SEARCH, vol. 3, no. 5, 1971, pages 285-295;T.
YOSHIMOTO et al.: "Studies on bacterial
proteases"

EXPERIENTA, vol. 43, no. 10, 1987, pages
1110-1111; H. SUMI et al.: "A novelfibrinolytic
enzyme (nattokinase) in the vegetable
cheese Natto; a typical andpopular soybean
food in the Japanese diet"

(73) Proprietor: NIHON CHEMICAL RESEARCH
KABUSHIKI KAISHA also known as JAPAN
CHEMICAL RESEARCH CO., LTD
4-20, Mikagehommachi 3-chome
Higashinada-ku
Kobe Hyogo 658 (JP)

(72) Inventor: Nakanishi, Koichiro
19-20, Karibadai 2-chome,
Nishi-ku
Kobe, Hyogo 673 (JP)
Inventor: Nomura, Keiichi
18-6, Toyoura-cho
Higashiosaka, Osaka 579 (JP)
Inventor: Tajima, Kyoko
Parumezon-Imadera 307,
4-1, Imadera,
Nishi-ku
Kobe, Hyogo 673 (JP)
Inventor: Hiratani, Hajime
705-3, Tottori,
Hannan-cho
Sennan-gun, Osaka 599-02 (JP)
Inventor: Kato, Kazuo
Howaito Rejidensu 202,
8-15, Minamigoyo 3-chome
Kita-ku, Kobe, Hyogo 651-11 (JP)

PATENT ABSTRACTS OF JAPAN, vol. 10, no. 366 (C-390)[2423], 6th December 1986;& JP-A-61 162 184

CHEMICAL ABSTRACTS, vol. 93, no. 9, 1st September 1980, page 246, abstract no.90793h, Columbus, Ohio, US; K.I. FAYEK et al.: "Purification and properties ofa fibrinolytic enzyme from Bacillus subtilis",& Z. ALLG. MIKROBIOL. 1980, 20(6), 375-82

(74) Representative: **Smart, Peter John et al**
**W.H. BECK, GREENER & CO**
**7 Stone Buildings**
**Lincoln's Inn**
**London WC2A 3SZ (GB)**

## Description

This invention relates to a protein in high purity, having fibrinolytic activity even when administered orally and a production method therefor.

Thrombus has come to be a serious problem as a cause of various diseases such as peripheral arteriovenous thrombosis, pulmonary embolism, mycocardial infarction, cardiac infarction, cerebral infarction, retinal arteriovenous thrombosis, or many other diseases. Sumi et al. have filed a patent application for a method of obtaining a fibrinolytic enzyme produced by Bacillus natto (Patent Application Kokai No. 162184/1986). Furthermore, Hiratani et al. filed another application (Patent Application Kokai No. 180834/1989) about a thrombolytic agent for oral administration comprising the above enzyme, whereby the efficacy of the enzyme was clarified. According to these applications, the above enzyme is white powder having molecular weight of about 20,000.

However, the purity of the fibrinolytic enzyme in these publications was low and hence its essential feature could not be clarified and a large amount, such as 1 g per adult dog, of administration was required to demonstrate the activity.

The present inventors have succeeded in obtaining substantially pure fibrinolytic protein from Natto (a Japanese food prepared by fermenting steamed soybeans in straw) or the culture of Bacillus natto.

In the first place, this invention is explained briefly.

The protein of this invention is contained in Natto, Bacillus natto or the culture of Bacillus natto, therefore, the protein can be obtained by extracting these materials with an aqueous solvent such as water or aqueous solution of salt(s), and then purifying the aqueous extract with a combination of at least two of fractionation means, for example, fractionation with alcohol, salting out with ammonium sulfate, hydrophobic chromatography employing a carrier such as Butyl-Sepharose® (produced by Pharmacia Co.) etc., anion exchange chromatography employing a carrier such as Mono-Q or S-Sepharose® (produced by Pharmacia Co.), gel-filtration chromatography employing Sephacryl® S-200 (produced by Pharmacia Co.), etc. In order to obtain the enzyme in high purity, hydrophobic chromatography is particularly effective.

The present inventors carried out researches into the highly purified protein thus obtained, and as a result, succeeded in clarifying the whole of its physico-chemical properties. Furthermore, it was found that this substance exhibits fibrinolytic activity even in a small dose of about 20 mg per adult dog, which led to the establishment of this invention.

This invention is directed to a protein which comprises the polypeptide having the amino acid sequence represented by the formula,

```
                        10                  20                        30
A Q S V P Y G I S Q I K A P A L H S Q G Y T G S N V K V A V

I D S G I D S S H P D L N V R G G A S F V P S E T N P Y G D

G S S H G T H V A G T I A A L N N S I G V L G V A P S A S L

Y A V K V L D S T G S G Q Y S W I I N G I E W A I S N N M D

V I N M S L G G P T G S T A L K T V V D K A V S S G I V V A

A A A G N E G S S G S T S T V G Y P A K Y P S T I A V G A V

N S S N Q R A S F S S V G S E L D V M A P G V S I Q S T L P

G G T Y G A Y N G T S M A T P H V A G A A A L I L S K H P T

W T N A Q V R D R L E S T A T Y L G N S F Y Y G K G L I N V

Q A A A Q
```

and having the following specific properties:

(a) Molecular weight: 31,000 (SDS-PAGE),
(b) Isoelectric point: 8.5 - 8.9,
(c) Fibrinolytic activity, and
(e) Hydrolytic activity to the synthetic substrate of Suc-Ala-Ala-Pro-Phe-MCA, and

a method for producing a fibrinolytic protein characterized by subjecting the aqueous extract of Natto or the culture of Bacillus natto to fractionation with alcohol and/or salting out, purifying the resulting insoluble material by chromatography including at least hydrophobic chromatography, and recovering a fraction containing the protein as described above.

The protein of this invention can be advantageously extracted from Natto or the culture of Bacillus natto. The aqueous extract of Natto or the culture of Bacillus natto can be obtained by extracting the material with water or neutral or weakly basic aqueous solution of salt(s). The liquid portion of a culture obtained by cultivating Bacillus natto in an aqueous medium may be employed as the aqueous extract.

The aqueous solution of salt(s) employed in the extraction includes, for example, a phosphate buffer (pH 6-8) containing a salt such as 0.01 M - 0.3 M of sodium chloride or potassium chloride, or 0.005 M - 0.1 M tris(hydroxymethyl)aminomethane (herein after abbreviated as "tris") buffer (pH 7-9) containing 0.01 M - 0.3 M of sodium chloride or potassium chloride.

There may be obtained a crude precipitate fraction by adding an organic solvent or a salt to the above crude extract. For example, the crude precipitate fraction can be obtained by adding to the aqueous extract an alcohol such as ethanol or methanol so as to make its concentration 60% - 80% (v/v), preferably 75 %, or ammonium sulfate so as to make its concentration 40 - 60% (w/v), preferably 50% (w/v).

The fraction of crude precipitates can be further purified by carrying out chromatography mentioned below;

The crude fraction is adsorbed on a carrier such as Butyl Sepharose® or Alkyl Sepharose® (produced by Pharmacia Co.), etc. which has hydrophobic groups and has been equilibrated with a neutral or weakly basic buffer containing sodium sulfate, and then eluted from the carrier with water or neutral or weakly basic buffer, whereby the fraction can be extracted and purified.

For example, as the solvent in the adsorption step, there may by employed a phosphate buffer (pH 6-8) containing 1.3-3 M of sodium sulfate or a tris-buffer (pH 7-8) containing the same amount of sodium sulfate as above.

As a different method for purification, co-exsisting impurities in the crude fraction can be removed by adsorbing the fraction on an anion exchanger equilibrated with a neutral or weakly basic buffer.

As the anion exchanger, there may be employed, for example, Mono-Q-Sepharose® (wherein particle distribution is extremely narrow, produced by Pharmacia Co.) or Q-Sepharose® Fast Flow (wherein the cross-linking ratio of Sepharose is increased to fortify the material physically and chemically, produced by Pharmacia Co.).

Furthermore, it can be purified by allowing it to adsorb on a cation exchanger equilibrated with a neutral or weakly basic buffer solution and then by elution with a neutral or weakly basic buffer solution containing salt(s).

As the carrier for the adsorption, there may be employed a cation exchanger such as S-Sepharose® First-Flow (produced by Pharmacia Co.) equilibrated with a buffer such as 0.005 M - 0.05 M phosphate buffer solution (pH 6-8) etc.

The elution from the carrier may be carried out by employing a buffer solution such as 0.005 M to 0.05 M phosphate buffer (pH 6-8) containing 0.2 to 1 M, preferably 0.4 to 0.6 M of sodium chloride, or 0.005 M to 0.05 M tris-buffer (pH 7-9) etc.

Furthermore, the enzyme of present invention may be purified by applying it on to a gel filtration carrier equilibrated with a neutral or weakly basic buffer solution. As the gel filtration carrier, there may be employed Sephacryl S-200 or Sephadex G-75 etc. For the equilibration of the carrier, there may be employed a buffer solution such as 0.005 M to 0.05 M phosphate buffer solution (pH 6-8) containing 0.05 to 0.5 M, preferably 0.2 M, sodium chloride or 0.005 M to 0.05 M tris-buffer solution (pH 7-9) etc.

Present fibrinolytic active protein can be obtained in high yield by employing these chromatographies in a combination of at least two of them.

The protein thus obtained can dissolve thrombus or prevent the formation of thrombus by oral administeration in an amount of 20 to 500 mg, preferably 50 to 300 mg at a time or dividing to several times daily to an adult.

As mentioned above, present invention provides a harmless and highly pure protein exhibiting fibrinolytic activity by oral administration in small amount, which is usable as a fibrinolytic agent for oral administration.

In the following, present protein is described in detail:

The protein of present invention comprises the structure of a peptide having the amino acid sequence represented by the formula,

4

```
                    10                   20                   30
A Q S V P Y G I S Q I K A P A L H S Q G Y T G S N V K V A V
I D S G I D S S H P D L N V R G G A S F V P S E T N P Y G D
G S S H G T H V A G T I A A L N N S I G V L G V A P S A S L
Y A V K V L D S T G S G Q Y S W I I N G I E W A I S N N M D
V I N M S L G G P T G S T A L K T V V D K A V S S G I V V A
A A A G N E G S S G S T S T V G Y P A K Y P S T I A V G A V
N S S N Q R A S F S S V G S E L D V M A P G V S I Q S T L P
G G T Y G A Y N G T S M A T P H V A G A A A L I L S K H P T
W T N A Q V R D R L E S T A T Y L G N S F Y Y G K G L I N V
Q A A A Q ,
```

and has the following specific properties;

(a) Molecular weight: 31,000 (SDS-PAGE),
(b) Isoelectric pH: 8.5 - 8.9,
(c) Fibrinolytic activity, and
(e) Hydrolytic activity to a synthetic substrate.

The respective properties mentioned above were determined by the methods described in the following Experiment a) to d).

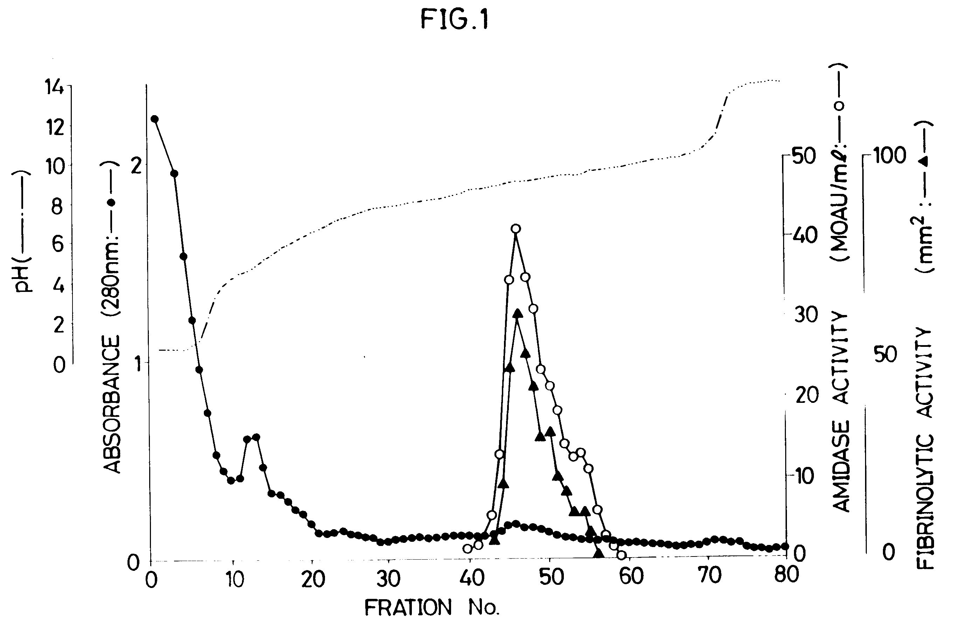

Referring to the drawing, Fig. 1 shows the result obtained by the isoelectric fractionation of present protein in Experiment b).

Experiment

a) Method of determining molecular weight:

To a sample of protein prepared by the method described in Example 1, SDS (sodium dodecyl sulfate) and beta-mercaptoethanol were added and the mixture was boiled at 100° C for 3 minutes.

The sample thus reacted with SDS was subjected to SDS-electrophoresis (SDS-PAGE), making use of an electrophoresis apparatus (Phast System® produced by Pharmacia Co.), employing 12.5 % acrylamide gel (Phast Gel Homogeneous$^R$, produced by Pharmacia Co.) and SDS buffer strip.

Staining was carried out by employing a silver stain kit (Silver Stain Kanto®, produced by Kanto Chemical Co.). Making use of a kit of determining molecular weight by electrophoresis (HMW kit E (for low molecular weight)), calibration curve was prepared and the molecular weight of the protein was determined.

The protein of present invention exhibited single stain band at molecular weight 31,000.

b) Method of determining isoelectric point:

A sugar density gradient wherein density inclines larger from upper layer to lower layer of a column was prepared by piling up Ampholine® (pH 3.5-10.0; produced by Pharmacia Co.) on a solution of sugar in Ampholine® (pH 7.0-9.0), employing isoelectric focusing electrophoresis apparatus (110 ml, produced by Kato Seisakusho). On this procedure, the protein prepared by the method of Example 1 was piled at the center of the column. A pH gradient was prepared by charging electricity to the column at 500 V for 43 hours.

By taking out solution in order from the bottom layer, there were obtained 80 fractions each having gradually increased pH. The amount of protein (absorbance at 280 nm) and pH as well as fibrinolytic activity and amidase activity of each fraction were determined.

The results are shown in Fig. 1. The isoelectric point of the protein of present invention was 8.5-8.9.

c) Evaluation of activity with fibrin plate (fibrinolytic activity):

Fibrin standard plate (T. Astrup and S. Mullertz, Archs. Biochem. Biophys., 40, 346-351, 1952) was prepared and 10 $\mu$l of the protein prepared by the method of Example 1 was spotted on the plate. After maintaining the plate at 37° C for 18 hours, the dissolved area ($mm^2$) of each fibrin film was measured. The dissolved area of fibrin with 4-fold dilution of the protein was 240.3 $mm^2$/ml.

d) Evaluation of amidase activity by employing Suc-Ala-Ala-Pro-Phe-MCA substrate:

The amidase activity of the protein prepared by the method of Example 1 was determined employing Suc-Ala-Ala-Pro-Phe-MCA produced by Peptide Kenkyusho.

After dissolving Suc-Ala-Ala-Pro-Phe-MCA in dimethylsufoxide (DMSO), the solution was added to 0.05 M tris-HCl buffer solution (pH 8.0) containing 0.15 M of sodium chloride to make 10 mM solution of the sustrate. The protein was suitably diluted with 0.05 M tris-HCl buffer solution (pH 8.0) containing 0.15 M of sodium chloride to make sample solutions. To 1 ml of the substrate solution being maintained at 37° C, 0.1 ml of the sample solution was added and allowed to react at 37° C for 30 minutes. The reaction was stopped by adding 4 ml of 17 % acetic acid thereto. Employing a fluorophotometer produced by Shimadzu Corp., the amount of 4-methylcoumarin (AMC) formed by the hydrolysis of Suc-Ala-Ala-Pro-Phe-MCA was calculated by comparing the strength of 460 nm fluorescence emitted from the sample under exciting by 380 nm light with that from a known amount of AMC. One unit of amidase activity (1 MCAU) was represented by the amount of enzyme which produces 1 nM of AMC per minute by hydrolyzing Suc-Ala-Ala-Pro-Phe-MCA.

The substrate-hydrolyzing activity of the protein obtained by the method of Example 1 was 107.6 MCAU/ml.

e) Amino acid composition:

220 pM of the protein obtained by the method of Example 1 was hydrolyzed in a sealed vacuum tube with 6 N hydrochloric acid containing 1 % of phenol at 110° C for 24, 48 or 72 hours, respectively, and then, employing an amino acid analyzer (Model 6300, produced by Beckmann Co.), the amino acid composition of each hydrolyzed protein was analyzed. Values of 0 hour were extrapolated regarding serine and threonine, and values at 72 hours were employed regarding valine, isoleucine and leucine. Furthermore, regarding tryptophan, its value was obtained by hydrolyzing with 3 N mercaptoethanesulfonic acid. Amino acid analysis after oxidation with performic acid revealed that the protein contains no cystine (and cysteine). The amino acid composition of the protein in pure state and amino acid sequence described below in Example 1 could not be revealed until obtaining the protein in high purity by the method developed under intensive research in the course of establishing present invention.

Table 1 shows the result of the amino acid analysis, wherein it is clear that the protein exhibits high homology with some enzymes of subtilisin family, particularly subtilisin amylosacchariticus.

Table 1. Amino acid composition of the protein
of present invention

| Amino acid | Present protein | Subtilisin | | |
|:---:|:---:|:---:|:---:|:---:|
| | | Carsberg | BPN' | Amilosacchariticus |
| Asx | 25 | 28 | 28 | 25 |
| Thr | 19 | 19 | 13 | 17 |
| Ser | 39 | 32 | 37 | 41 |
| Glx | 15 | 12 | 15 | 15 |
| Pro | 13 | 9 | 14 | 13 |
| Gly | 33 | 35 | 33 | 33 |
| Ala | 34 | 41 | 37 | 35 |
| Val | 28 | 31 | 30 | 25 |
| Met | 4 | 5 | 5 | 4 |
| Ile | 16 | 10 | 13 | 16 |
| Leu | 15 | 18 | 15 | 15 |
| Tyr | 12 | 13 | 10 | 12 |
| Phe | 3 | 4 | 3 | 3 |
| Lys | 8 | 9 | 11 | 8 |
| His | 6 | 5 | 6 | 6 |
| Arg | 4 | 4 | 2 | 4 |
| Cys | 0 | 0 | 0 | 0 |
| Trp | 3 | 1 | 3 | 3 |
| Total | 275 | 274 | 275 | 275 |

Present invention is described more concretely by the examples referred below:

Example 1

By extracting 20 kg of Natto obtained from the market with 35 L of physiological saline, crude extract containing 1.8 MCAU/mg of protein was obtained. Ethanol was added to the extract to make 50 % ethanol solution which was then centrifuged, and, to the supernatant, further ethanol was added to procipitate 936 g of a fraction with 75 % ethanol. This precipitate fraction was dissolved in 0.01 M PB (phosphate buffer solution, pH 7.0) containing 1.5 M of ammonium sulfate, adsorbed on Butyl-Sepharose® (produced by Pharmacia Co.) equilibrated with the same buffer solution, eluted from the carrier with 0.01 M PB (pH 7.0), and then salted out from the eluate by adding 50 % w/v ammonium sulfate thereto to obtain 1085 g of a protein fraction (193 MCAU/mg). Purification efficiency in the above processes of treatment with Butyl-Sepahrose and subsequent salting out with ammonium sulfate was 20.3 times, and the rate of recovering activity by the end of the process was 42.5 %.

The protein fraction was subjected to desalting and concentration, and passed through 1 ml column of anion exchanger, Mono-Q® (produced by Pharmacia Co.), equilibrated with tris-HCl (pH 7.0) to obtain 706 mg of a protein fraction (204 MCAU/mg). This fraction was subjected to the hydrophobic chromatography consisted of dissolving the fraction in 0.01 M PB (pH 7.0) containing 1.5 M of ammonium sulfate, adsorbing

7

on 1 ml of Alkyl-Superose® (produced by Pharmacia Co.) equilibrated with the same PB, and eluting from the carrier with 0.01 M PB (pH 7.0) to obtain 277 mg of protein (390 MCAU/mg). The recovery rate of activity of total processes was 22.0 %. The properties and purity of this fraction are shown in the previous Experiment concerning properties.

The purified protein was digested to fragments with lysyl-endopeptidase and the fragments were purified by reverse HLPC to obtain 9 fractions. The amino acid sequence of each fraction was determined by applying the fraction to Model 477A/120A produced by Applied Biosystem Co. If necessary, each peptide fragment was further digested with chymotrypsin and purified, and then the sequence was determined.

The results are shown in Table 2. The mutating sites of present protein to subtilisin amilosacchariticus were 4 positions of $^{130}S \rightarrow ^{130}T$, $^{102}S \rightarrow ^{102}T$, $^{192}A \rightarrow ^{192}V$ and $^{259}D \rightarrow ^{259}N$.

## Table 2. Total Amino acid sequence
## of present protein

```
                10                    20                    30
A Q S V P Y G I S Q I K A P A L H S Q G Y T G S N V K V A V
                40                    50                    60
I D S G I D S S H P D L N V R G G A S F V P S E T N P Y G D
                70                    80                    90
G S S H G T H V A G T I A A L N N S I G V L G V A P S A S L
                100                   110                   120
Y A V K V L D S T G S G Q Y S W I I N G I E W A I S N N M D
                130                   140                   150
V I N M S L G G P T G S T A L K T V V D K A V S S G I V V A
                160                   1701                  180
A A A G N E G S S G S T S T V G Y P A K Y P S T I A V G A V
                190                   200                   210
N S S N Q R A S F S S V G S E L D V M A P G V S I Q S T L P
                220                   230                   240
G G T Y G A Y N G T S M A T P H V A G A A A L I L S K H P T
                250                   260                   270
W T N A Q V R D R L E S T A T Y L G N S F Y Y G K G L I N V
          275
Q A A A Q
```

### Example 2

From 20 kg of Natto, 630 mg of crude protein was obtained in the same manner as in Example 1 until elution from Butyl-Sepharose and purified by the following procedures;

The crude protein was adsorbed on S-Sepharose Fast Flow® (produced by Pharmacia Co.) equilibrated with 0.05 M phosphate buffer solution (pH 7.0), eluted from the carrier with 0.05 M phosphate buffer solution (pH 7.0) containing 0.5 M of sodium chloride and further purified employing Sephacryl® S-200 (produced by Pharmacia Co.) equilibrated with 10 mM phosphate buffer solution containing 0.2 M of sodium chloride to obtain 257 mg of purified protein fraction (379 MCAU/mg). Recovering rate of activity through total processes was 18.0 %.

### Example 3

A culture of Bacillus natto (100 ml) which had been pre-cultured in nutrient agar medium was inoculated in 10,000 ml of Henneberg's liquid and cultured at 43 to 45 °C for 45 hours. The liquid culture of Bacillus natto was then centrifuged at 1,850 x g for 10 minutes, and the resulting supernatant was subjected to

8

salting out by adding 50 % (w/v) of ammonium sulfate thereto to obtain 35 g of crude product.

The crude product was purified by the processes of treatment with Butyl-Sepharose, Mono-Q and alkyl-Superose in order according to Example 1 to obtain 53 mg of purified protein fraction (310 MCAU/mg protein). Total recovering rate of activity was 18.9 %.

Example 4 (Activity)

In the lateral latent vein of monogrel adult dogs (male, 10-16 kg), artificial thrombi were prepared employing 0.4 mg of 5 % bovine fibrinogen and 50 units/ml of bovine thrombin by the method of Sasaki et al (K. Sasaki et al., Life Science, 27, 1659-1685, 1980). Four enteric capsules containing 10 mg/capsule of the protein produced by the method described in Example 1 were administered at one time to each dog. After the capsules moved to the duodenum, blood was collected from the dog and euroglobulin-dissolving time was measured by the above Sasaki et al's method as well as angiography by X ray was carried out after 4 ml of Angiografin® (produced by Schering, West Germany) was injected taking 2 seconds.

As the results, it was found that the contrast group (N = 3) to which capsules not containing present protein were administered did not exhibit variation in ELT for 30 minutes to 12 hours and the dissolving of the artificial thrombus was not observed. To the contrary, in the group to which present protein was administered, ELT was $33 \pm 6$ minutes ($p < 0.02$) at 30 minutes after the administration, $42 \pm 10$ minutes ($p < 0.09$) after 1 hour, $52 \pm 8$ minutes ($p < 0.5$) after 3 hours and $53 \pm 8$ minutes ($p < 0.5$) after 6 hours, showing shortening (i.e. acceleration in fibrinolysis). Furthermore, it was confirmed that the artificial thrombi were completely dissolved within 5 hours of ELT in the group to which present protein was administered.

From the above facts, it is apparent that present protein exhibits fibrinolytic activity at an extremely small amount as compared with an effective dose, not less than 1 g, of known crude protein obtained from Bacillus natto.

SEQ ID NO:1
SEQUENCE TYPE: Amino acid
SEQUENCE LENGTH: 275

MOLECULE TYPE: Protein
HYPOTHETICAL? No
FRAGMENT TYPE: Unspecified
ORIGINAL SOURCE
OGANISM: Natto or <u>bacillus</u> <u>natto</u>
PROPERTY: Fibrinolytic and thrombolytic

```
Ala Gln Ser Val Pro Tyr Gly Ile Ser Gln Ile Lys Ala Pro Ala
1               5                   10                  15
Leu His Ser Gln Gly Tyr Thr Gly Ser Asn Val Lys Val Ala Val
                20                  25                  30
Ile Asp Ser Gly Ile Asp Ser Ser His Pro Asp Leu Asn Val Arg
                35                  40                  45
Gly Gly Ala Ser Phe Val Pro Ser Glu Thr Asn Pro Tyr Gly Asp
                50                  55                  60
Gly Ser Ser His Gly Thr His Val Ala Gly Thr Ile Ala Ala Leu
                65                  70                  75
Asn Asn Ser Ile Gly Val Leu Gly Val Ala Pro Ser Ala Ser Leu
                80                  85                  90
Tyr Ala Val Lys Val Leu Asp Ser Thr Gly Ser Gly Gln Tyr Ser
                95                  100                 105
Trp Ile Ile Asn Gly Ile Glu Trp Ala Ile Ser Asn Asn Met Asp
                110                 115                 120
Val Ile Asn Met Ser Leu Gly Gly Pro Thr Gly Ser Thr Ala Leu
                125                 130                 135
Lys Thr Val Val Asp Lys Ala Val Ser Ser Gly Ile Val Val Ala
                140                 145                 150
Ala Ala Ala Gly Asn Glu Gly Ser Ser Gly Ser Thr Ser Thr Val
                155                 160                 165
Gly Tyr Pro Ala Lys Tyr Pro Ser Thr Ile Ala Val Gly Ala Val
                170                 175                 180
```

```
Asn Ser Ser Asn Gln Arg Ala Ser Phe Ser Ser Val Gly Ser Glu
                185                 190                 195
Leu Asp Val Met Ala Pro Gly Val Ser Ile Gln Ser Thr Leu Pro
                200                 205                 210
Gly Gly Thr Tyr Gly Ala Tyr Asn Gly Thr Ser Met Ala Thr Pro
                215                 220                 225
His Val Ala Gly Ala Ala Ala Leu Ile Leu Ser Lys His Pro Thr
                230                 235                 240
Trp Thr Asn Ala Gln Val Arg Asp Arg Leu Glu Ser Thr Ala Thr
                245                 250                 255
Tyr Leu Gly Asn Ser Phe Tyr Tyr Gly Lys Gly Leu Ile Asn Val
                260                 265                 270
Gln Ala Ala Ala Gln
                275
```

**Claims**

**Claims for the following Contracting States : CH, DE, FR, GB, IT, LI**

1.  A protein which comprises the polypeptide having the amino acid sequence represented by the formula,

```
                        10                  20                  30
A Q S V P Y G I S Q I K A P A L H S Q G Y T G S N V K V A V
I D S G I D S S H P D L N V R G G A S F V P S E T N P Y G D
G S S H G T H V A G T I A A L N N S I G V L G V A P S A S L
Y A V K V L D S T G S G Q Y S W I I N G I E W A I S N N M D
V I N M S L G G P T G S T A L K T V V D K A V S S G I V V A
A A A G N E G S S G S T S T V G Y P A K Y P S T I A V G A V
N S S N Q R A S F S S V G S E L D V M A P G V S I Q S T L P
G G T Y G A Y N G T S M A T P H V A G A A A L I L S K H P T
W T N A Q V R D R L E S T A T Y L G N S F Y Y G K G L I N V
Q A A A Q
```

and having the following specific properties:
    (a) Molecular weight: 31,000 (SDS-PAGE),
    (b) Isoelectric point: 8.5 - 8.9,
    (c) Fibrinolytic activity, and
    (e) Hydrolytic activity to the synthetic substrate Suc-Ala-Ala-Pro-Phe-MCA.

2.  A fibrinolytic enzyme which comprises a protein as claimed in Claim 1.

3.  A pharmaceutical or veterinary preparation comprising a protein as claimed in Claim 1, formulated for pharmaceutical or veterinary use.

4. A thrombolytic agent for oral administration which comprises a protein as described in Claim 1.

5. A method for producing a protein as claimed in Claim 1, comprising extracting said protein from natto or a culture of Bacillus natto.

6. A method as claimed in Claim 5, characterised by subjecting an aqueous extract of Natto or a culture of Bacillus natto to fractionation with alcohol and/or salting out, purifying the resulting insoluble material by chromatography including at least hydrophobic chromatography, and recovering a fraction containing the protein.

7. A method as described in Claim 6, wherein the chromatography includes hydrophobic chromatography and anion exchange chromatography.

8. A method as claimed in Claim 7, wherein the chromatography further includes gel filtration chromatography.

9. The use of a protein as claimed in Claim 1, in the manufacture of a medicament for the prophylactic or therapeutic treatment of thrombus and thrombus related conditions.

**Claims for the following Contracting State : ES**

1. A method for producing a protein which comprises the polypeptide having the amino acid sequence represented by the formula,

```
                    10                    20                    30
  A Q S V P Y G I S Q I K A P A L H S Q G Y T G S N V K V A V
  I D S G I D S S H P D L N V R G G A S F V P S E T N P Y G D
  G S S H G T H V A G T I A A L N N S I G V L G V A P S A S L
  Y A V K V L D S T G S G Q Y S W I I N G I E W A I S N N M D
  V I N M S L G G P T G S T A L K T V V D K A V S S G I V V A
  A A A G N E G S S G S T S T V G Y P A K Y P S T I A V G A V
  N S S N Q R A S F S S V G S E L D V M A P G V S I Q S T L P
  G G T Y G A Y N G T S M A T P H V A G A A A L I L S K H P T
  W T N A Q V R D R L E S T A T Y L G N S F Y Y G K G L I N V
  Q A A A Q
```

and having the following specific properties:
   (a) Molecular weight: 31,000 (SDS-PAGE),
   (b) Isoelectric point: 8.5 - 8.9,
   (c) Fibrinolytic activity, and
   (e) Hydrolytic activity to the synthetic substrate of Suc-Ala-Ala-Pro-Phe-MCA,
comprising extracting said protein from natto or a culture of Bacillus natto.

2. A method as claimed in Claim 1 for producing a fibrinolytic protein characterised by subjecting an aqueous extract of Natto or a culture of Bacillus natto to fractionation with alcohol and/or salting out, purifying the resulting insoluble material by chromatography including at least hydrophobic chromatography, and recovering a fraction containing the protein.

3. A method as described in Claim 2, wherein the chromatography includes hydrophobic chromatography and anion exchange chromatography.

4. A method as claimed in Claim 3, wherein the chromatography further includes gel filtration chromatography.

5. The use of a protein which comprises the polypeptide having the amino acid sequence represented by the formula,

```
                        10                      20                      30
A Q S V P Y G I S Q I K A P A L H S Q G Y T G S N V K V A V
I D S G I D S S H P D L N V R G G A S F V P S E T N P Y G D
G S S H G T H V A G T I A A L N N S I G V L G V A P S A S L
Y A V K V L D S T G S G Q Y S W I I N G I E W A I S N N M D
V I N M S L G G P T G S T A L K T V V D K A V S S G I V V A
A A A G N E G S S G S T S T V G Y P A K Y P S T I A V G A V
N S S N Q R A S F S S V G S E L D V M A P G V S I Q S T L P
G G T Y G A Y N G T S M A T P H V A G A A A L I L S K H P T
W T N A Q V R D R L E S T A T Y L G N S F Y Y G K G L I N V
Q A A A Q
```

and having the following specific properties:
(a) Molecular weight: 31,000 (SDS-PAGE),
(b) Isoelectric point: 8.5 - 8.9,
(c) Fibrinolytic activity, and
(e) Hydrolytic activity to the synthetic substrate Suc-Ala-Ala-Pro-Phe-MCA
in the manufacture of a medicament for the prophylactic or therapeutic treatment of thrombus and thrombus related conditions.

**Patentansprüche**
**Patentansprüche für folgende Vertragsstaaten : CH, DE, FR, GB, IT, LI**

1. Protein, das das Polypeptid mit der nachfolgend dargestellten Aminosäuresequenz

```
                        10                      20                      30
A Q S V P Y G I S Q I K A P A L H S Q G Y T G S N V K V A V
I D S G I D S S H P D L N V R G G A S F V P S E T N P Y G D
G S S H G T H V A G T I A A L N N S I G V L G V A P S A S L
Y A V K V L D S T G S G Q Y S W I I N G I E W A I S N N M D
V I N M S L G G P T G S T A L K T V V D K A V S S G I V V A
A A A G N E G S S G S T S T V G Y P A K Y P S T I A V G A V
N S S N Q R A S F S S V G S E L D V M A P G V S I Q S T L P
G G T Y G A Y N G T S M A T P H V A G A A A L I L S K H P T
W T N A Q V R D R L E S T A T Y L G N S F Y Y G K G L I N V
Q A A A Q
```

umfaßt und die folgenden spezifischen Eigenschaften besitzt:
(a) Molekulargewicht: 31.000 (SDS-PAGE),
(b) isoelektrischer Punkt: 8,5 - 8,9,

(c) fibrinolytische Aktivität, und

(e) hydrolytische Aktivität gegenüber dem synthetischen Substrat Suc-Ala-Ala-Pro-Phe-MCA.

2. Fibrinolytisches Enzym, das ein Protein gemäß Anspruch 1 umfaßt.

3. Pharmazeutisches oder veterinärärztliches Präparat, das ein Protein gemäß Anspruch 1 umfaßt und zur pharmazeutischen oder veterinärärztlichen Verwendung formuliert ist.

4. Thrombolytisches Agens zur oralen Verabreichung, das ein Protein gemäß Anspruch 1 umfaßt.

5. Verfahren zur Herstellung eines Proteins gemäß Anspruch 1, welches das Extrahieren des Proteins aus Natto oder einer Kultur von Bacillus natto umfaßt.

6. Verfahren nach Anspruch 5, dadurch gekennzeichnet, daß man einen wäßrigen Extrakt von Natto oder einer Kultur von Bacillus natto einer Fraktionierung mit Alkohol und/oder Aussalzen unterzieht, das resultierende unlösliche Material mittels Chromatographie einschließlich mindestens einer hydrophoben Chromatographie reinigt, und man eine das Protein enthaltende Fraktion gewinnt.

7. Verfahren gemäß Beschreibung in Anspruch 6, bei dem die Chromatographie eine hydrophobe Chromatographie und eine Anionenaustauschchromatographie einschließt.

8. Verfahren nach Anspruch 7, bei dem die Chromatographie ferner eine Gelfiltrationschromatographie einschließt.

9. Verwendung eines Proteins gemäß Anspruch 1 bei der Herstellung eines Medikaments zur prophylaktischen oder therapeutischen Behandlung eines Thrombus und damit zusammenhängenden Erkrankungen.

**Patentansprüche für folgenden Vertragsstaat : ES**

1. Verfahren zur Herstellung eines Proteins, das das Polypeptid mit der nachfolgend dargestellten Aminosäuresequenz

```
              10                    20                    30
A Q S V P Y G I S Q I K A P A L H S Q G Y T G S N V K V A V
I D S G I D S S H P D L N V R G G A S F V P S E T N P Y G D
G S H G T H V A G T I A A L N N S I G V L G V A P S A S L
Y A V K V L D S T G S G Q Y S W I I N G I E W A I S N N M D
V I N M S L G G P T G S T A L K T V V D K A V S S G I V V A
A A A G N E G S S G S T S T V G Y P A K Y P S T I A V G A V
N S S N Q R A S F S S V G S E L D V M A P G V S I Q S T L P
G G T Y G A Y N G T S M A T P H V A G A A A L I L S K H P T
W T N A Q V R D R L E S T A T Y L G N S F Y Y G K G L I N V
Q A A A Q
```

umfaßt und die folgenden spezifischen Eigenschaften besitzt:

(a) Molekulargewicht: 31.000 (SDS-PAGE),

(b) isoelektrischer Punkt: 8,5 - 8,9,

(c) fibrinolytische Aktivität, und

(e) hydrolytische Aktivität gegenüber dem synthetischen Substrat Suc-Ala-Ala-Pro-Phe-MCA,

umfassend das Extrahieren des Proteins aus Natto oder einer Kultur von Bacillus natto.

2. Verfahren nach Anspruch 1 zur Herstellung eines fibrinolytischen Proteins, dadurch gekennzeichnet, daß man einen wäßrigen Extrakt von Natto oder einer Kultur von <u>Bacillus</u> <u>natto</u> einer Fraktionierung mit Alkohol und/oder Aussalzen unterzieht, das resultierende unlösliche Material mittels Chromatographie einschließlich mindestens einer hydrophoben Chromatographie reinigt, und man eine das Protein enthaltende Fraktion gewinnt.

3. Verfahren gemäß Beschreibung in Anspruch 2, bei dem die Chromatographie eine hydrophobe Chromatographie und eine Anionenaustauschchromatographie einschließt.

4. Verfahren nach Anspruch 3, bei dem die Chromatographie ferner eine Gelfiltrationschromatographie einschließt.

5. Verwendung eines Proteins, das das Polypeptid mit der nachfolgend dargestellten Aminosäuresequenz

```
                  10              20              30
A Q S V P Y G I S Q I K A P A L H S Q G Y T G S N V K V A V
I D S G I D S S H P D L N V R G G A S F V P S E T N P Y G D
G S S H G T H V A G T I A A L N N S I G V L G V A P S A S L
Y A V K V L D S T G S G Q Y S W I I N G I E W A I S N N M D
V I N M S L G G P T G S T A L K T V V D K A V S S G I V V A
A A A G N E G S S G S T S T V G Y P A K P S T I A V G A V
N S S N Q R A S F S S V G S E L D V M A P G V S I Q S T L P
G G T Y G A Y N G T S M A T P H V A G A A A L I L S K H P T
W T N A Q V R D R L E S T A T Y L G N S F Y Y G K G L I N V
Q A A A Q
```

umfaßt und die folgenden spezifischen Eigenschaften besitzt:
  (a) Molekulargewicht: 31.000 (SDS-PAGE),
  (b) isoelektrischer Punkt: 8,5 - 8,9,
  (c) fibrinolytische Aktivität, und
  (e) hydrolytische Aktivität gegenüber dem synthetischen Substrat Suc-Ala-Ala-Pro-Phe-MCA,
zur Herstellung eines Medikaments zur prophylaktischen oder therapeutischen Behandlung eines Thrombus und damit zusammenhängenden Erkrankungen.

**Revendications**
**Revendications pour les Etats contractants suivants : CH, DE, FR, GB, IT, LI**

1. Protéine qui contient le polypeptide ayant la séquence d'acides aminés représentée par la formule

```
                    10                  20                  30
A Q S V P Y G I S Q I K A P A L H S Q G Y T G S N V K V A V
I D S G I D S S H P D L N V R G G A S F V P S E T N P Y G D
G S S H G T H V A G T I A A L N N S I G V L G V A P S A S L
Y A V K V L D S T G S G Q Y S W I I N G I E W A I S N N M D
V I N M S L G G P T G S T A L K T V V D K A V S S G I V V A
A A A G N E G S S G S T S T V G Y P A K Y P S T I A V G A V
N S S N Q R A S F S S V G S E L D V M A P G V S I Q S T L P
G G T Y G A Y N G T S M A T P H V A G A A A L I L S K H P T
W T N A Q V R D R L E S T A T Y L G N S F Y Y G K G L I N V
Q A A A Q
```

et ayant les propriétés spécifiques suivantes:
   (a) poids moléculaire : 31 000 (SDS-PAGE),
   (b) point isoélectrique : 8,5 - 8,9,
   (c) activité fibrinolytique, et
   (d) activité hydrolytique sur le substrat synthétique Suc-Ala-Ala-Pro-Phe-MCA.

2. Enzyme fibrinolytique qui contient une protéine selon la revendication 1.

3. Préparation pharmaceutique ou vétérinaire comprenant une protéine selon la revendication 1, formulée pour un usage pharmaceutique ou vétérinaire.

4. Agent thrombolytique pour administration par voie orale qui contient une protéine telle que décrite dans la revendication 1.

5. Procédé de production d'une protéine selon la revendication 1, qui consiste à extraire ladite protéine du natto ou d'une culture de *Bacillus natto*.

6. Procédé selon la revendication 5, caractérisé en ce qu'on soumet un extrait aqueux de natto ou une culture de *Bacillus natto* à un fractionnement avec de l'alcool et/ou un relargage, on purifie le matériau insoluble résultant par chromatographie incluant au moins une chromatographie hydrophobe et on recueille une fraction contenant la protéine.

7. Procédé selon la revendication 6, dans lequel la chromatographie inclut la chromatographie hydrophobe et la chromatographie d'échange d'ions.

8. Procédé selon la revendication 7, dans lequel la chromatographie inclut en outre la chromatographie de filtration sur gel.

9. Utilisation d'une protéine selon la revendication 1, dans la fabrication d'un médicament pour le traitement prophylactique ou thérapeutique du thrombus ou de troubles apparentés au thrombus.

**Revendications pour l'Etat contractant suivant : ES**

1. Procédé de production d'une protéine qui contient le polypeptide ayant la séquence d'acides aminés représentée par la formule

```
                         10                    20                      30
A Q S V P Y G I S Q I K A P A L H S Q G Y T G S N V K V A V
I D S G I D S S H P D L N V R G G A S F V P S E T N P Y G D
G S S H G T H V A G T I A A L N N S I G V L G V A P S A S L
Y A V K V L D S T G S G Q Y S W I I N G I E W A I S N N M D
V I N M S L G G P T G S T A L K T V V D K A V S S G I V V A
A A A G N E G S S G S T S T V G Y P A K Y P S T I A V G A V
N S S N Q R A S F S S V G S E L D V M A P G V S I Q S T L P
G G T Y G A Y N G T S M A T P H V A G A A A L I L S K H P T
W T N A Q V R D R L E S T A T Y L G N S F Y Y G K G L I N V
Q A A A Q
```

et ayant les propriétés spécifiques suivantes:
(a) poids moléculaire : 31 000 (SDS-PAGE),
(b) point isoélectrique : 8,5 - 8,9,
(c) activité fibrinolytique, et
(d) activité hydrolytique sur le substrat synthétique Suc-Ala-Ala-Pro-Phe-MCA,
qui consiste à extraire ladite protéine du natto ou d'une culture de *Bacillus natto*.

2. Procédé selon la revendication 1 pour produire une protéine fibrinolytique, caractérisé en ce qu'on soumet un extrait aqueux de natto ou une culture de *Bacillus natto* à un fractionnement avec de l'alcool et/ou un relargage, on purifie le matériau insoluble résultant par chromatographie incluant au moins une chromatographie hydrophobe et on recueille une fraction contenant la protéine.

3. Procédé selon la revendication 2, dans lequel la chromatographie inclut la chromatographie hydrophobe et la chromatographie d'échange d'ions.

4. Procédé selon la revendication 3, dans lequel la chromatographie inclut en outre la chromatographie de filtration sur gel.

5. Utilisation d'une protéine qui contient le polypeptide ayant la séquence d'acides aminés représentée par la formule

```
                    10                      20                      30
A Q S V P Y G I S Q I K A P A L H S Q G Y T G S N V K V A V
I D S G I D S S H P D L N V R G G A S F V P S E T N P Y G D
G S S H G T H V A G T I A A L N N S I G V L G V A P S A S L
Y A V K V L D S T G S G Q Y S W I I N G I E W A I S N N M D
V I N M S L G G P T G S T A L K T V V D K A V S S G I V V A
A A A G N E G S S G S T S T V G Y P A K Y P S T I A V G A V
N S S N Q R A S F S S V G S E L D V M A P G V S I Q S T L P
G G T Y G A Y N G T S M A T P H V A G A A A L I L S K H P T
W T N A Q V R D R L E S T A T Y L G N S F Y Y G K G L I N V
Q A A A Q
```

et ayant les propriétés spécifiques suivantes:
  (a) poids moléculaire : 31 000 (SDS-PAGE),
  (b) point isoélectrique : 8,5 - 8,9,
  (c) activité fibrinolytique, et
  (d) activité hydrolytique sur le substrat synthétique Suc-Ala-Ala-Pro-Phe-MCA
dans la fabrication d'un médicament pour le traitement prophylactique ou thérapeutique du thrombus
ou de troubles apparentés au thrombus.

# FIG.1

FRATION No.